## Europäisches Patentamt
## European Patent Office
## Office européen des brevets

(19)

(11) Publication number: **0 331 640**
**A2**

(12)

# EUROPEAN PATENT APPLICATION

(21) Application number: **89810155.5**

(51) Int. Cl.⁴: $A\ 61\ K\ 37/02$

(22) Date of filing: **28.02.89**

(30) Priority: **04.03.88 GB 8805231**
**09.05.88 GB 8810899**

(43) Date of publication of application:
**06.09.89 Bulletin 89/36**

(84) Designated Contracting States: **ES GR LU**

(71) Applicant: **SANDOZ AG**
**Lichtstrasse 35**
**CH-4002 Basel (CH)**

(72) Inventor: **Feyen, Jean Honoré M.**
**Mühlegasse 2**
**CH-4104 Oberwil (CH)**

**Trechsel, Ulrich**
**Bollwerkstrasse 60**
**CH-4102 Binningen (CH)**

The title of the invention has been amended (Guidelines for Examination in the EPO, A-III, 7.3).

(54) **Use of an interleukin-6 molecule in inhibiting bone resorption.**

(57) Interleukin-6 molecules are useful in inhibiting bone resorption and increasing bone formation.

**EP 0 331 640 A2**

**Description**

## BONE RESORPTION

This invention relates to bone resorption.

Interleukin 6 is a cytokine which has been shown to be produced by fibroblasts and many other cell types. Production of interleukin 6 and its activity on, e.g. bone marrow is described in PCT application PCT/US 87/01611 filed off US Appln No. 06/885905 filed July 15, 1986 ( WO 88/00206). Interleukin 6 appears to have multiple target cells. Interleukin 6 turned out to be identical to interferon beta-2, B-cell stimulatory factor 2 (BSF-2), interleukin HP-1 and 26K factor. It is now doubtful whether interleukin-6 can be regarded as an interferon at all in view of its low anti-viral activity. It has been reported that the production of interleukin 6 can be stimulated by interleukin 1 (IL-1) and Tumour Necrosis Factor (TNFalpha) in certain cells. It is also known that prostaglandin $E_2$ production, a marker of bone resorption, is stimulated by IL-1 and TNFalpha.

Despite intensive research with interleukin-6 in particular immunological and hematological areas, there has been no report of effects of interleukin 6 on bone resorption or formation.

We have now found that IL-6 molecules (as defined hereinafter) are useful in inhibiting bone resorption and increasing bone formation. They are thus useful in treating osteoporosis, e.g. acute and/or chronic stages of postmenopausal osteoporosis, including primary and/or secondary osteoporosis and localized osteoporosis, post traumatic osteoporosis, Paget's disease of bone (osteitis deformans), osteolysis due to bone malignancies, e.g. osteogenic sarcoma, osteodystrophy, e.g. renal osteodystrophy, osteomalacia, multiple myeloma of the bone, osteogenesis imperfecta, hypercalcemia, osteomyelitis, e.g. in conjunction with antibiotics and ectopic calcification.

The results below show that IL-6 molecules (as defined hereinafter):

i) inhibit $PGE_2$ secretion from bone cell cultures (Test 1)

ii) inhibit $PGE_2$ secretion stimulation induced by e.g. two lymphokines, interleukin-1 and tumour necrosis factor, in bone cultures (Test 2),

iii) increase bone formation (Test 3), and

iv) decrease bone resorption (Test 4 and Test 5).

The increase in bone formation is of particular interest.

Additionally it is shown that osteoblasts, which also mediate osteoclasts, produce interleukin-6 activity (Tests 6 and 7).

As used hereinafter 1 unit (U) of interleukin-6 activity refers to the amount of IL-6 molecule which causes half-maximal stimulation in the B-cells proliferation assay e.g. using clone B13.29 (which is freely available from the Central Laboratory Netherlands Red Cross, Blood Transfusion Service, P.O.Box 9406, 1006 AK Amsterdam) as described in the specific assay of Landsdorp et al. Curr.Top. Microbiol. Immunol. (1986) 132, 105. and Aarden et al. Eur. J. Immunol. (1987) 17, 1411. 1 x $10^8$ units is about 1 mg of polypeptide.

In brief, cells are suspended in culture medium (Iscove's modified Dulbecco's medium supplemented with 5% (v/v) fetal calf serum, penicillin and streptomycin) and plated (5.$10^3$ cells /200 uL medium) in microtiter plates. Cells are cultured for 48 hours, in a humidified atmosphere of 5% $CO_2$ in air at 37°C, the last 6 hours in the presence of 1 microCi of [$^3$H] thymidine and the radioactivity incorporated in the nuclei analysed.

The conditioned medium to be tested is serial diluted in B13.29 cell medium.

Interleukin-6 for which test data is given refers to glycosylated IL-6 produced by CHO cells, if desired purification to remove LPS (lipopolysaccharide).

As used hereinafter interleukin-1 alpha = IL-1.

Test 1

Basal production of prostaglandin $E_2$ in the rat osteosarcoma cell line ROS 17/2.8 is inhibited at a concentration of from about 0.01 to about 100 U/ml IL-6 molecule. In this test cells of the rat osteosarcoma cell line ROS 17/2.8 are cultured in Dulbecco's minimal essential medium and medium F12 (1:1) containing 10% fetal calf serum and 2 mM L-glutamine in a humidified atmosphere of 5% $CO_2$ in air at 37°C. At confluency, cells are treated with the above concentration of IL-6 molecule. After 24 hours of culture an aliquot of the medium is assayed for prostaglandin $E_2$ using a standard radioimmunoassay.

Results obtained with interleukin-6 are as follows:-

| Concentration of interleukin-6 in U/ml | $PGE_2$ Pg (picograms)/ml |
|---|---|
| 0 (Control) | 184 |
| 0.1 | 151 |
| 1.0 | 128 |
| 10 | 118 |

Test 2

Production of prostaglandin $E_2$ produced by stimulation of mouse calvarial cells by interleukin-1 and tumor necrosis factor is reduced at a concentration of from about 10 to about 100 Units/ml of IL-6 molecule.

In this test organ cultures of mouse calvariae are first prepared:-

Frontal and parietal bones are dissected from neonatal (5 to 6-day-old) mice (strain CD-1), split along the sagittal suture, and cultured in 2 ml of BGJ- medium containing 1 mg/ml bovine serum albuminin, penicillin and streptomycin in 35 mm plastic tissue culture wells.

After the preculture period (24 hours), the medium is replaced with BGJ-culture medium supplemented with interleukin-1 at a concentration of 10 units or TNFalpha at a concentration of 10 nanograms/ml and the IL-6 molecule at the above concentration and cultured for another 48 hours. During the entire culture period, the multi-well dishes are shaken on a rocker platform (15 oscillations/min) in a humidified atmosphere of 5% $CO_2$ in air at 37°C. The calvaria-conditioned medium is harvested and placed at 4°C.

An aliquot of the medium is then assayed for prostaglandin $E_2$ using a radioimmunoassay.

Results obtained with interleukin-6 are as follows:-

Stimulation by 10 U/ml interleukin-1

| Concentration of Interleukin-6 (Units/ml) | Prostaglandin $E_2$ pg/ml |
|---|---|
| 0 | 4800 |
| 10 | 4400 |
| 100 | 500* |

(* equivalent to control value without stimulation by interleukin-6).

Stimulation by 10 ng/ml TNFa

| Concentration of Interleukin-6 (Units/ml) | Prostaglandin $E_2$ pg/ml |
|---|---|
| 0 | 3300 |
| 10 | 1200 |
| 100 | 800* |

(* Control value without stimulation = 500 pg/ml)

If prostaglandin $E_2$ secretion stimulation is induced by the non-lymphokine parathyroid hormone ($10^{-8}$ molar) then a small inhibition of prostaglandin synthesis is still observed.

| Concentration of Interleukin-6 (Units/ml) | Prostaglandin $E_2$ pg/ml |
|---|---|
| 0 | 3700 |
| 10 | 3450 |
| 100 | 3300 |

Interleukin-6 has no effect on prostaglandin $E_2$ synthesis of unstimulated bone cultures in the above test.

Test 3

IL-6 molecules increase bone formation as indicated by e.g. an increased incorporation of [3H]proline into collagen and non-collagen proteins in fetal rat calvaria.

Organ cultures of rat calvaria are prepared by dissecting frontal and pariatal bones from 21-day old fetal rats, splitting along the sagittal suture and culturing according to the method of Kream et al. (Endocrinology (1985) 116, 296). The IL-6 molecules are added in doses of from 1 to 100 units to 2 ml of cultures. Culturing is effected for 24 to 48 hours.

To quantitate the incorporation of [3H]proline into collagenase-digestable protein and non-collagen protein, bone homogenates are digested with bacterial collagenase according to the method of Diegelmann R. and Peterkofsky (Dev.Biol. (1972) 28:443) and modified by Kream et al. (Endocrinology (1985) 116:296).

3

Test 4

11-6 molecules decrease bone resorption as indicated by a decrease in release of [45]Ca from bone.

The test is effected according to the principles of Raisz (J.Clin.Invest. (1965) 44, 103).

Pregnant rats are injected s.c. with [45]Ca on the eighteenth day of gestation. Placebo or IL-6 molecules (1 to 100 Units per animal) are injected s.c. On day nineteen, the animals are sacrified, the fetuses removed. The mineralized shafts of the radii and ulnae are dissected and placed in culture. Resorption is quantitated on the basis of release of [45]Ca from the bone explants.

Test 5

IL-6 molecules decrease bone resorption as indicated by a decreased number of osteoclasts and an increased amount of bone as indicated in the following test:-

Growing mice (6 to 8 weeks old; weight ca. 20 grams) are used. IL-6 molecules in doses of from about 0.1 to about 10 micrograms, e.g. 1 microgram, are injected subcutaneously over the calvarial bones, e.g. over the frontal and temporal bones of the skull. Injections are made daily for three to ten days. 3 days, 7 days or 2 weeks after the cessation of treatment the animals are sacrified. The calvarial bones are examined by histology. For example the frontal and temporal bones are fixed, embedded and sectioned. The decrease in bone resorption relative to control animals is observed in a morphological basis, e.g. by more bone matrix and by a decreased number of osteoclasts as revealed by staining e.g. with tartrate resistant acid phosphatase.

Moreover, an increase in bone formation in the calvarial bones, e.g. frontal and temporal bones, relative to control animals is observed on a morphological basis, e.g. by an increased bone growth, and also observed by an increase in the number of osteoblasts.

Test 6

Release of interleukin-6 like activity is stimulated in human osteoblast-like Saos-2 cells by phorbol 12-myristate 13-acetate (PMA) (which is a known stimulator of interleukin-6 from non-bone cell cultures) (at a concentration 2 to 200 of nanogram/ml), interleukin-1 (1 to 20 units/ml) and parathyroid hormone (PTH) ($10^{-7}$ to $10^{-10}$ M).

In this test the osteoblastic human osteosarcoma cell line Saos-2 is cultured in McCoy's 5A medium supplemented 15% (v/v) fetal calf serum (FCS), penicillin, streptomycin and 2 mM L-glutamine in a humidified atmosphere of 5% $CO_2$ in air at 37°C. At confluency, cells are refed with medium containing 2 or 10% fetal calf serum and supplemented with the different agents to be tested. The medium of the Saos-2 cells is harvested after a 48 hour or 72 hour stimulation period and stored at 4°C.

Interleukin-6 like activity is detected using a conventional tritium -thymidine incorporation assay as follows:-

Conditioned medium of Soas-2 cells are assayed for the presence of interleukin-6 activity using the above-mentioned B-cell (clone B13.29) proliferation assay. Significant levels of interleukin-6 are detected within 48 hours.

Representative results are as follows:-

FCS = 2%

Dilution of conditional medium 1/10

| Stimulator | $^3$H-thymidine incorporation (cpm)* |
|---|---|
| None | 874 |
| PMA (20 mg/ml) | 61015 |
| IL-1 (10 U/ml) | 23189 |
| PTH ($10^{-8}$ M) | 1287 |

* (background = 400 cpm; maximum incorporation 66000 cpm)

No detectable amounts of interleukin-6 like activity under control non-stimulated conditions are observed.

Test 7

Culturing of the mouse calvarial cells described above under test 2 gives significant interleukin-6 activity under control conditions (20000 cpm) after 48 hours culture. This is stimulated in the presence of phorbol 12-myristate 13-acetate (PMA) (1 to 100 ng /ml) and parathyroid hormone, hPTH$_{1-34}$, (PTH) ($10^{-7}$ to $10^{-10}$ M).

Results obtained ($^3$H thymidine incorporation in cpm) are:-

|   | Dilution | Control | PMA 100 ng/ml | PMA 10 ng/ml | PMA 1 ng/ml |
|---|----------|---------|---------------|--------------|-------------|
| 1 | 10       | 39919   | 47510         | 37679        | 48150       |
| 2 | 100      | 9075    | 48239         | 36162        | 18145       |
| 3 | 1000     | 680     | 27694         | 17687        | 1370        |
| 4 | 10000    | 346     | 5885          | 2545         | 580         |
| 5 | 100000   | 305     | 595           | 572          | 411         |

|   | PTH 10-7 M | 7 PTH 10-8 M | 8 PTH 10-9 M | 9 PTH 10-10 M |
|---|------------|--------------|--------------|---------------|
| 1 | 12326      | 14638        | 16252        | 35897         |
| 2 | 23222      | 26885        | 30172        | 13073         |
| 3 | 22816      | 18503        | 9059         | 827           |
| 4 | 1935       | 1279         | 1095         | 531           |
| 5 | 336        | 452          | 682          | 678           |

The present invention provides accordingly

i) a method of inhibiting bone resorption or increasing bone formation or treating osteoporosis or osteoarthritis or any other indication mentioned above in a subject which comprises administering a therapeutically effective amount of an IL-6 molecule to a subject in need of such treatment,

ii) the use of an IL-6 molecule in the manufacture of a medicament suitable for inhibiting bone resorption or increasing bone formation or treating osteoporosis or osteoarthritis or any other indication mentioned above,

iii) the use of an IL-6 molecule for inhibiting bone resorption or increasing bone formation or treating osteoporosis or osteoarthritis or any other indication mentioned above, or

iv) a medicament for inhibiting bone resorption or increasing bone formation or treating osteoporosis or osteoarthritis or any other indication mentioned above containing an IL-6 molecule. By IL-6 molecule is meant any compound corresponding to, having essentially the same structure as, or the same spectrum of activity as the known interleukin-6 (see the above mentioned PCT application or in the specific proliferation assay referrred to above with reference to the Landsdorp and Aarden publications ) or factors mentioned above, e.g. interferon beta-2 as described in e.g. EP 220574A (Yeda), PCT application WO 88/00206 (Genetics Institute), and EP 257406 (Ajinomoto). The contents of all these publications (hereinafter interleukin-6 publications) are incorporated herein by reference.

The IL-6 molecule may be glycosylated, e.g. on the argninine sites, and may be produced, e.g. from E.coli or CHO cells.

The IL-6 molecule as described is a 212 amino acid polypeptide. If desired the first or last methionine amino acid or even the whole non-coding region may be omitted, i.e. the first 28 amino acids, from Met.Asn up to and including Phe Pro Ala.

The interleukin-6 may have one, two or three or more amino acids absent, added or replaced by others and still retain interleukin-6 like activity, and be active in the method of the invention.

The expression IL-6 molecule covers such analogues as well as any compound which selectively stimulates IL-6 activity in bone cultures, especially if the compound is selective in that it has an immaterial action on other factors or bone resorption by other routes.

The term thus includes a compound of formula I:-

PRO VAL PRO PRO GLY GLU ASP SER LYS ASP VAL

ALA ALA PRO HIS ARG GLN PRO LEU THR SER SER

GLU ARG ILE ASP LYS GLN ILE ARG TYR ILE LEU

ASP GLY ILE SER ALA LEU ARG LYS GLU THR CYS

ASN LYS SER ASN MET CYS GLU SER SER LYS GLU

ALA LEU ALA GLU ASN ASN LEU ASN LEU PRO LYS

MET ALA GLU LYS ASP GLY CYS PHE GLN SER GLY

PHE ASN GLU GLU THR CYS LEU VAL LYS ILE ILE

THR GLY LEU LEU GLU PHE GLU VAL TYR LEU GLU                I

TYR LEU GLN ASN ARG PHE GLU SER SER GLU GLU

GLN ALA ARG ALA VAL GLN MET SER THR LYS VAL

LEU ILE GLN PHE LEU GLN LYS LYS ALA LYS ASN

LEU ASP ALA ILE THR THR PRO ASP PRO THR THR

ASN ALA SER LEU LEU THR LYS LEU GLN ALA GLN

ASN GLN TRP LEU GLN ASP MET THR THR HIS LEU

ILE LEU ARG SER PHE LYS GLU PHE LEU GLN SER

SER LEU ARG ALA LEU ARG GLN MET.

It also includes a compound of formula I having additional amino acids, e.g. attached to the N-terminal group, e.g. Ala or Met Ala or of formula II.

ALA PRO THR SER SER SER THR LYS LYS THR GLN

LEU GLN LEU GLU HIS LEU LEU LEU ASP LEU PHE                (II)

ARG ALA – X

wherein X is formula I.

The term also includes molecules wherein one or more amino acids are replaced or omitted, e.g. incorporating the sequence.

PRO VAL PRO PRO GLY GLU ASP SER LYS

ASP VAL ALA ALA

e.g. as the N-terminal sequence, and/or retaining essentially the same sequence of the remainder.

For these indications, the appropriate dosage will, of course, vary depending upon, for example, exact IL-6 molecule employed, the host, the mode of administration and the nature and severity of the condition being treated. However, in general, satisfactory results in animals are indicated to be obtained at daily dosages from about 0.5 micrograms/kg to about 20 micrograms/kg animal body weight. In larger mammals, for example humans, an indicated daily dosage is in the range from about 25 micrograms to about 1000 micrograms of an IL-6 molecule conveniently administered, for example, in divided doses up to four times a day.

The IL-6 molecules may be administered by any conventional route, in particular enterally e.g. in the form of tablets or capsules, or preferably parenterally, e.g. in the form of injectable solutions or suspensions.

For example methods of administration and galenical formulations for use interleukin-6 are known, e.g. from the above-mentioned interleukin-6 publications.

Interleukin-6 is the preferred compound. Test results are given above. It is indicated that the compound may be administered at daily dosages of from 25 micrograms to 125 micrograms by subcutaneous injection to larger mammals, for example humans.

Pharmaceutical compositions may comprise an IL-6 molecule in association with at least one pharmaceutical carrier or diluent. Such compositions may be manufactured in conventional manner. Unit dosage forms contain, for example, from about 4 micrograms to about 500 micrograms of the IL-6 molecule.

The present composition also provides a pack containing a pharmaceutical composition comprising an IL-6 molecule together with instructions for use in a method as defined above.

**Claims**

1. The use of an IL-6 molecule in the manufacture of a medicament suitable for inhibiting bone resorption.

2. The use of an IL-6 molecule in the manufacture of a medicament suitable for increasing bone formation.

3. The use of an IL-6 molecule in the manufacture of a medicament suitable for treating osteoporosis.

4. The use of an IL-6 molecule in the manufacture of a medicament suitable for treating osteoarthritis.

5. The use of an IL-6 molecule in the manufacture of a medicament suitable for treating at least one of the following diseases; Paget's disease of bone, osteolysis due to bone malignancies, osteomalacia, osteogenesis imperfecta and osteomyelitis.

6. The use of an IL-6 molecule in the manufacture of a medicament suitable for treating multiple myeloma of the bone.

7. The use of an IL-6 molecule in the manufacture of a medicament suitable for treating hypercalcemia.

8. A use according to any preceeding claim wherein the IL-6 molecule is interleukin-6.

9. A use according to any preceeding claim wherein the IL-6 molecule is of formula I:-

```
PRO VAL PRO PRO GLY GLU ASP SER LYS ASP VAL

ALA ALA PRO HIS ARG GLN PRO LEU THR SER SER

GLU ARG ILE ASP LYS GLN ILE ARG TYR ILE LEU

ASP GLY ILE SER ALA LEU ARG LYS GLU THR CYS

ASN LYS SER ASN MET CYS GLU SER SER LYS GLU

ALA LEU ALA GLU ASN ASN LEU ASN LEU PRO LYS

MET ALA GLU LYS ASP GLY CYS PHE GLN SER GLY

PHE ASN GLU GLU THR CYS LEU VAL LYS ILE ILE

THR GLY LEU LEU GLU PHE GLU VAL TYR LEU GLU          I

TYR LEU GLN ASN ARG PHE GLU SER SER GLU GLU

GLN ALA ARG ALA VAL GLN MET SER THR LYS VAL

LEU ILE GLN PHE LEU GLN LYS LYS ALA LYS ASN

LEU ASP ALA ILE THR THR PRO ASP PRO THR THR

ASN ALA SER LEU LEU THR LYS LEU GLN ALA GLN

ASN GLN TRP LEU GLN ASP MET THR THR HIS LEU

ILE LEU ARG SER PHE LYS GLU PHE LEU GLN SER

SER LEU ARG ALA LEU ARG GLN MET.
```

10. A use according to claim 8 wherein the compound is Ala-X wherein X is the sequence of formula I.

11. A use according to claim 8 wherein the compound is Met-Ala-X wherein X is the sequence of formula I.

12. A use according to claim 9 wherein the IL-6 molecule incorporates the sequence

```
PRO VAL PRO PRO GLY GLU ASP SER LYS
ASP VAL ALA ALA.
```

13. A use according to claim 1 substantially as hereinbefore described with reference to any one of the examples.

14. A method of treating one of the diseases mentioned in any preceeding claim which comprises administering a compound as defined in any preceeding claim to a subject in need of such treatment.

15. A pack containing a pharmaceutical composition comprising an IL-6 molecule together with instructions for use in a method as defined in claim 14.